# EUROPEAN PATENT APPLICATION

(11) **EP 4 620 475 A1**
(43) Date of publication of application: **24.09.2025**
(21) Application number: 23903387.1
(22) Date of filing: 06.12.2023
(51) Int. Cl.: A61K 31/718, A23L 33/125, A61K 36/04, A61P 1/14

(54) **INTESTINAL ENVIRONMENT-IMPROVING AGENT**

(30) Priority: 12.12.2022 JP 2022197958
(71) Applicant: Galdieria Co., Ltd., Tokyo 103-0007 (JP)
(72) Inventor: ADAMS Eri, Tokyo 103-0007 (JP); MAEDA Kazuki, Tokyo 103-0007 (JP)
(74) Representative: Gilani, Anwar
(86) International application number: PCT/JP2023/043693
(87) International publication number: WO 2024/128098

(57) **Abstract**

The present invention relates to an agent for improving intestinal environment, containing, as an active ingredient, a glycogen derived from *Galdieria sulphuraria.*

## Description

### Technical Field

The present invention relates to an agent for improving intestinal environment.

### Background Art

Glycogen is a polymer having a branched structure in which α-D-glucose is polymerized with an α-(1 → 4) bond or an α-(1 → 6) bond. Glycogen is known as a stored polysaccharide in prokaryotes and animals. Glycogen greatly differs in molecular weight and proportion of branched structure depending on the type of organism from which it is derived. Glycogen derived from *Galdieria sulphuraria,* a kind of red algae, is known to have a high proportion of branched structures (Non Patent Literature 1).

### Citation List

### Non Patent Literature

Non Patent Literature 1: Carbohydrate Polymers, 2017, Vol. 169, pages 75 to 82

### Summary of Invention

### Technical Problem

The present inventors have found that the α-diversity index of the intestinal bacterial flora increases and the proportion of bifidobacteria and butyric acid bacteria, which are known as good bacteria, in the intestinal bacterial flora increases in humans who ingest the glycogen derived from *G*. *sulphuraria.* The present invention is based on this novel finding, and an object thereof is to provide a novel agent for improving intestinal environment.

### Solution to Problem

One aspect of the present invention relates to an agent for improving intestinal environment, containing, as an active ingredient, glycogen derived from *Galdieria sulphuraria.*

As described above, the glycogen derived from *G*. *sulphuraria* has a newly found attribute of increasing the α-diversity index of the intestinal bacterial flora and increasing the proportion of bifidobacteria and butyric acid bacteria, which are known as good bacteria, in the intestinal bacterial flora in humans who ingest the glycogen. Therefore, the glycogen derived from *G*. *sulphuraria* can be suitably used in applications for improving the intestinal environment.

The agent for improving intestinal environment according to the present invention can also be regarded as an agent for increasing α-diversity of the intestinal bacterial flora, an agent for increasing the proportion of bifidobacteria in the intestinal bacterial flora, or an agent for increasing the proportion of butyric acid bacteria in the intestinal bacterial flora.

### Advantageous Effects of Invention

According to the present invention, a novel agent for improving intestinal environment can be provided.

### Brief Description of Drawings

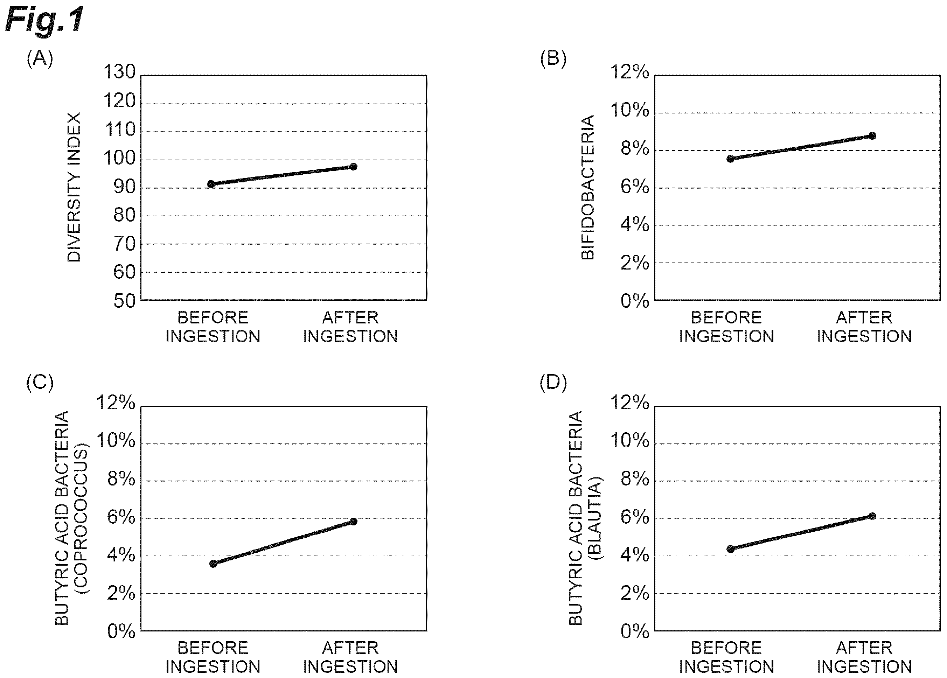

FIG. 1 is a graph showing the results of analyzing intestinal bacterial flora before and after ingestion in humans (n = 5) who ingested glycogen derived from *G*. *sulphuraria.* (A) A graph showing a result of analyzing the α-diversity index of the intestinal bacterial flora. (B) A graph showing a result of analyzing a proportion of bifidobacteria in the intestinal bacterial flora. (C) A graph showing the results of analyzing the proportion of butyric acid bacteria (bacteria belonging to the genus *Coprococcus*) in the intestinal bacterial flora. (D) A graph showing a result of analyzing a proportion of butyric acid bacteria (bacteria belonging to the genus *Blautia*) in the intestinal bacterial flora.

### Description of Embodiments

Hereinafter, embodiments for carrying out the present invention will be described in more detail. However, the present invention is not limited to the following embodiments.

The agent for improving intestinal environment according to the present embodiment contains, as an active ingredient, glycogen derived from *Galdieria sulphuraria.*

*G. sulphuraria* is a red alga belonging to the order *Cyanidiales.* Glycogen derived from *G*. *sulphuraria* is known to have a high proportion of branched structures (Non Patent Literature 1). As described in the Examples below, the present invention is characterized in that the glycogen derived from *G*. *sulphuraria* is used for an application for improving the intestinal environment based on the findings that the glycogen derived from *G*. *sulphuraria* increases the α-diversity index of the intestinal bacterial flora and increases the proportion of bifidobacteria and butyric acid bacteria, which are known as good bacteria, in the intestinal bacterial flora.

As the glycogen derived from *G*. *sulphuraria,* those extracted or purified from *G*. *sulphuraria* can be used. The method for extracting or purifying the glycogen from *G*. *sulphuraria* is not particularly limited, and for example, the glycogen can be obtained by the method described in Non Patent Literature (International Journal of Biological Macromolecules, 2016, Vol. 89, pp. 12-18). In addition, for example, as described in Examples described later, the glycogen can also be obtained by extracting glycogen from a dried powder of *G*. *sulphuraria* with water, then removing contaminants such as proteins in the extract (for example, removal by heat treatment or removal by TCA precipitation), and purifying the glycogen by ethanol precipitation. As the *G*. *sulphuraria* for extracting or purifying the glycogen, those cultured by a conventional method can be used.

The agent for improving intestinal environment according to the present embodiment may be composed only of a glycogen as the active ingredient derived from *G*. *sulphuraria,* or may contain, in addition to the active ingredient, other ingredients acceptable as a pharmaceutical product, a quasi-pharmaceutical product, or a food. The content of the glycogen as an active ingredient derived from *G*. *sulphuraria* in the agent for improving intestinal environment according to the present embodiment is not particularly limited, and may be, for example, in a range of 0.01 mass% or more and 100 mass% or less with respect to the total amount of the agent for improving intestinal environment.

Examples of the other components include, but are not limited to, an excipient, a binder, a lubricant, a disintegrant, an emulsifier, a surfactant, a base, a solubilizing agent, and a suspending agent.

Examples of the excipient include lactose, sucrose, starch, and dextrin. Examples of the binder include polyvinyl alcohol, gum arabic, tragacanth, gelatin, hydroxypropylmethylcellulose, hydroxypropylcellulose, sodium carboxymethylcellulose, and polyvinylpyrrolidone. Examples of the lubricant include magnesium stearate, calcium stearate, and talc. Examples of the disintegrant include crystalline cellulose, agar, gelatin, calcium carbonate, sodium hydrogen carbonate, and dextrin. Examples of the emulsifier or surfactant include Tween 60, Tween 80, Span 80, and glyceryl monostearate. Examples of the base include cetostearyl alcohol, lanolin, polyethylene glycol, a rice bran oil, a fish oil (DHA, EPA, etc.), and an olive oil. Examples of the solubilizing agent include polyethylene glycol, propylene glycol, sodium carbonate, sodium citrate, and Tween 80. Examples of the suspending agent include Tween 60, Tween 80, Span 80, glyceryl monostearate, polyvinyl alcohol, polyvinylpyrrolidone, methyl cellulose, hydroxymethyl cellulose, and sodium alginate.

The agent for improving intestinal environment according to the present embodiment may have any shape such as a solid (for example, a powder obtained by freeze drying), a liquid (for example, a water-soluble or fat-soluble solution or suspension), or a paste.

The dosage form of the agent for improving intestinal environment according to the present embodiment may be, for example, any of a powder, a pill, a granule, a tablet, a syrup, a troche, a capsule, or an injection. When the dosage form is an injection, it may be, for example, any of an intravenous injection, a subcutaneous injection, an intramuscular injection, or an intraperitoneal injection.

The agent for improving intestinal environment according to the present embodiment can be used as a pharmaceutical ingredient, a food ingredient, a food additive, a feed ingredient, a feed additive, or the like. The food (including supplements) may be, for example, a food for specific health use, a food for special use, a nutritional supplement, or a functional food.

The agent for improving intestinal environment according to the present embodiment can be suitably used for an application for improving an intestinal environment because the glycogen derived from *G. sulphuraria* contained as the active ingredient increases at least the α-diversity index of the intestinal bacterial flora, and increases the proportion of bifidobacteria and butyric acid bacteria, which are known as good bacteria, in the intestinal bacterial flora.

The agent for improving intestinal environment according to the present embodiment can also be used for an application for increasing the α-diversity of the intestinal bacterial flora since the glycogen derived from *G*. *sulphuraria,* contained as the active ingredient, increases at least the α-diversity index of the intestinal bacterial flora. That is, the agent for improving intestinal environment according to the present embodiment can also be regarded as an agent for increasing α-diversity of the intestinal bacterial flora.

The agent for improving intestinal environment according to the present embodiment can be expected to exhibit an effect of preventing or ameliorating depression caused by stress, an effect of ameliorating inflammatory bowel disease, an effect of improving resistance to cancer, and an effect of preventing or ameliorating Alzheimer's disease via improving α-diversity of intestinal bacterial flora.

The agent for improving intestinal environment according to the present embodiment can also be suitably used for an application of increasing the proportion of bifidobacteria in the intestinal bacterial flora since the glycogen derived from *G*. *sulphuraria* contained as the active ingredient increases at least the proportion of bifidobacteria in the intestinal bacterial flora. That is, the agent for improving intestinal environment according to the present embodiment can also be regarded as an agent for increasing the proportion of bifidobacteria in the intestinal bacterial flora.

The agent for improving intestinal environment according to the present embodiment can be expected to exhibit an effect of suppressing inflammation, an effect of improving irritable bowel syndrome, an effect of alleviating symptoms of pollinosis, and a preventive effect or symptom alleviating effect on viral infections such as influenza by increasing the proportion of bifidobacteria in the intestinal bacterial flora.

The agent for improving intestinal environment according to the present embodiment can also be suitably used for an application of increasing the proportion of the butyric acid bacteria in the intestinal bacterial flora since the glycogen derived from *G*. *sulphuraria* contained as the active ingredient increases at least the proportion of the butyric acid bacteria in the intestinal bacterial flora. That is, the agent for improving intestinal environment according to the present embodiment can also be regarded as an agent for increasing the proportion of butyric acid bacteria in the intestinal bacterial flora.

The agent for improving intestinal environment according to the present embodiment can be expected to exhibit an effect of being able to enjoy a part of the beneficial effects exerted by exercise on the body, an effect of suppressing an excessive immune response to prevent diseases, an effect of reducing liver fat mass and inflammation, and an effect of increasing muscle mass, by increasing the proportion of butyric acid bacteria in the intestinal bacterial flora.

The dosage and administration when the agent for improving intestinal environment according to the present embodiment is administered to a subject can be appropriately set according to the type, condition, age, and the like of the subject. The subject may be a human or a non-human mammal. The subject is preferably a human. The administration method may be oral administration or parenteral administration. The administration method is preferably oral administration. An example of a specific dose is a dose at which the amount of the active ingredient is in the range of 0.1 g or more and 100 g or less per day. The dose is preferably in the range of an active ingredient amount of 1 g or more and 80 g or less per day, an active ingredient amount of 2 g or more and 60 g or less per day, an active ingredient amount of 3 g or more and 40 g or less per day, an active ingredient amount of 4 g or more and 30 g or less per day, an active ingredient amount of 5 g or more and 20 g or less per day, an active ingredient amount of 6 g or more and 15 g or less per day, an active ingredient amount of 7 g or more and 13 g or less per day, and an active ingredient amount of 8 g or more and 12 g or less per day. The agent for improving intestinal environment according to the present embodiment may be administered once a day, or may be administered in multiple divided doses such as twice a day, 3 times a day, or 4 or more times a day so that the amount of the active ingredient per day falls within the above-mentioned range.

The present invention can also be regarded as a glycogen derived from *Galdieria sulphuraria* for use in improving the intestinal environment. The present invention can further be regarded as a method for improving the intestinal environment, including administering an effective amount of the agent for improving intestinal environment containing glycogen derived from *Galdieria sulphuraria,* as an active ingredient, to a subject in need thereof. The present invention can also be regarded as the use of glycogen derived from *Galdieria sulphuraria* in the production of the agent for improving intestinal environment.

### Examples

Hereinafter, the present invention will be described more specifically based on Examples. However, the present invention is not limited to the following Examples.

### [Example 1: Preparation of Glycogen derived from G. sulphuraria]

Cells of *G*. *sulphuraria* 074w grown to stationary phase with 38.5 g/L sugar were recovered by serial centrifugation. The recovered cells were washed with water until pH reached around 5, dried at 37°C overnight, and then powdered using a milling machine to obtain a dried powder of *G*. *sulphuraria.*

600 mL of ultrapure water (MilliQ water) was added to 200 g of dried powder of *G*. *sulphuraria,* and the mixture was well stirred, then boiled in a water bath for 60 minutes. The boiled solution was cooled and then centrifuged (4,000 × g, RT, 10 min). After transferring the supernatant to a new centrifuge tube, 400 mL of ultrapure water (MilliQ water) was added to the pellet, and the mixture was stirred well, then centrifuged (4,000 × g, RT, 10 min), and further recovered glycogen. The supernatant obtained was combined with the supernatant obtained first.

1/10 volume of precooled 50% (wt/v) trichloroacetic acid (TCA) was then added to the supernatant to precipitate residual protein and the like. The mixture was allowed to stand on ice for 20 minutes, and then centrifuged (4,000 × g, 4°C, 20 min). The supernatant was transferred to a new centrifuge tube, 1.5 volumes of ethanol were added, and the tube was allowed to stand on ice for 30 minutes, and then centrifuged (4,000 × g, 4°C, 15 min) to precipitate the glycogen.

The supernatant was then discarded and the pellet was resuspended in 300 mL ultrapure water (MilliQ water) warmed to about 70°C. 1.5 volumes of ethanol were added to this, and it was left to stand on ice for 60 minutes, the mixture was centrifuged (4,000 × g, 4°C, 30 min) to precipitate glycogen. The supernatant was discarded and the pellet was scraped into a tray and dried at 75°C.

After transferring the dried pellets to a new centrifuge tube, the pellets were resuspended in 300 mL ultrapure water (MilliQ water) warmed to about 70°C. 1.5 volumes of ethanol were added to this, and it was left to stand on ice for 60 minutes, the mixture was centrifuged (4,000 × g, 4°C, 30 min)to precipitate glycogen. The supernatant was discarded and the pellet was scraped into a tray and dried at 75°C. The resulting dried pellet was taken as glycogen derived from *G*. *sulphuraria.*

### [Example 2: Effect of Improving Intestinal Environment by Glycogen derived from G. sulphuraria]

In 5 healthy human subjects (men and women in their 20s to 50s of age), glycogen derived from *G*. *sulphuraria* is orally ingested at a dose of 10 g/day before supper meal once a day. Feces before ingestion (In FIG. 1, "before ingestion") and feces after ingestion for 28 days (In FIG. 1, "after ingestion") were collected from each subject, and analysis of intestinal bacterial flora was performed.

The analysis of the intestinal bacterial flora was performed by meta 16S analysis. First, genomic DNA derived from the intestinal bacteria was extracted and purified from a specimen (feces). Next, the DNA sample was analyzed with a next generation sequencer (MiSeq, manufactured by Illumina) to obtain meta 16S nucleotide sequence data. The obtained nucleotide sequence data was collated with a database using Qiime2, which is a data platform of a next generation sequencer, thereby identifying the type of bacteria, obtaining the sequence number data of the bacteria type, and confirming the α-diversity index indicating the number of types of intestinal bacteria, the proportion of bifidobacteria (bacteria belonging to the genus Bifidobacterium), and the proportion of butyric acid bacteria (bacteria belonging to the genus *Coprococcus* and bacteria belonging to the genus *Blautia*) producing butyric acid, which is a kind of short-chain fatty acid. The results are shown in FIG. 1. The data in FIG. 1 is an average value of 5 subjects.

FIG. 1(A) is a graph showing a result of analyzing an α-diversity index of an intestinal bacterial flora. As shown in FIG. 1(A), by ingesting the glycogen derived from *G*. *sulphuraria,* the α-diversity index after the ingestion (after 28 days of ingestion) was increased more than that before the ingestion, and the α-diversity of the intestinal bacterial flora was improved. Hitherto, it has been reported that in a stress model mouse, functional depression of intestinal innate immunity caused by stress triggers abnormality of intestinal bacterial flora and disruption of intestinal metabolite homeostasis (Scientific Reports, 2021, Volume11, Article number 9915). It has been reported that a decrease in diversity of intestinal bacterial flora is observed in patients with inflammatory bowel disease (IBD) (Journal of the Society of Biological Engineers, 2021, Vol. 99, No. 11, pp. 584-586). It has been reported that in colorectal cancer patients, the diversity of intestinal bacteria in colorectal cancer tissues was lower than the diversity of normal tissues distant from cancer tissues (PLoS One. 2012; 7: e39743). In addition, it has been reported that the diversity of intestinal bacterial flora is low in patients with Alzheimer's disease, and the symptoms are alleviated by recovery of the diversity by probiotics (J. Appl. Microbiol., 2019, 127 (4), pp. 954-967). The agent for improving intestinal environment according to the present invention can be expected to exhibit an effect of preventing or ameliorating depression caused by stress, an effect of ameliorating inflammatory bowel disease, an effect of improving resistance to cancer, and an effect of preventing or ameliorating Alzheimer's disease via improving α-diversity of intestinal bacterial flora.

FIG. 1(B) is a graph showing a result of analyzing a proportion of bifidobacteria in the intestinal bacterial flora. As shown in FIG. 1(B), by ingesting the glycogen derived from *G*. *sulphuraria,* the proportion of bifidobacteria after the ingestion (after 28 days of ingestion) was increased more than that before the ingestion. Hitherto, it has been reported that inflammation is suppressed when a large amount of acetic acid produced by bifidobacteria exists in a model mouse of inflammatory disease (Eur. J. Immunol., 2007, 37 (8), pp. 2309-2316). It has been reported that when bifidobacteria (Bifidobacterium infantis) was administered to a human with irritable bowel syndrome (IBS), improvement in scores such as abdominal pain, bloating sensation, intestinal dysfunction, and defecation failure was observed (American Journal of Gastroenterology, 2006, 101 (7), pp. 1581-1590). It has been reported that when 44 subjects with pollinosis were caused to ingest bifidobacteria for 13 weeks in the season of pollinosis, symptoms of pollinosis such as a runny nose and nasal congestion were resolved (Clin. Exp. Allergy, 2006, 36 (11), pp. 1425-1435). In addition, it has been reported that the incidence rate of influenza was significantly lower in a group of elderly people who continuously ingested bifidobacteria (Bioscience Biotechnology and Biochemistry, 2010, Vol. 74, No. 5, pp. 939-945). The agent for improving intestinal environment according to the present invention can be expected to exhibit an effect of suppressing inflammation, an effect of improving irritable bowel syndrome, an effect of alleviating symptoms of pollinosis, and a preventive effect or symptom alleviating effect on viral infections such as influenza by increasing the proportion of bifidobacteria in the intestinal bacterial flora.

FIG. 1(C) is a graph showing the results of analyzing the proportion of butyric acid bacteria (bacteria belonging to the genus *Coprococcus*) in the intestinal bacterial flora. FIG. 1(D) is a graph showing a result of analyzing a proportion of butyric acid bacteria (bacteria belonging to the genus *Blautia*) in the intestinal bacterial flora. As shown in FIGS. 1(C) and (D), by ingesting the glycogen derived from *G. sulphuraria,* the proportion of butyric acid bacteria after the ingestion (after 28 days of ingestion) was increased more than that before the ingestion. Hitherto, it has been reported that when an exercise program was provided to a human, the fecal concentration of short-chain fatty acid (particularly butyric acid) increased, and then returned to a sedentary life for a certain period of time, the level of short-chain fatty acid decreased again (Med. Sci. Sports Exerc., 2018, 50 (4), pp. 747-757). It has been reported that butyric acid produced by intestinal bacteria changes immature T cells when absorbed in the intestinal tract and induces them into regulatory T cells that regulate an immune reaction, thereby suppressing an excessive immune response and contributing to prevention of diseases (Nature, 2013, Vol. 504, pp. 446-450). It has also been reported that when butyric acid bacteria (Faecalibacterium prausnitzii) were inoculated into mice on a high-fat diet, the fat content in the liver was reduced, inflammation was reduced, and the muscle mass was also increased (ISME J., 2017, 11 (7), pp. 1667-1679). The agent for improving intestinal environment according to the present invention can be expected to exhibit an effect of being able to enjoy a part of the beneficial effects exerted by exercise on the body, an effect of suppressing an excessive immune response to prevent diseases, an effect of reducing liver fat mass and inflammation, and an effect of increasing muscle mass, by increasing the proportion of butyric acid bacteria in the intestinal bacterial flora.

## Claims

1. An agent for improving intestinal environment, comprising, as an active ingredient, a glycogen derived from *Galdieria sulphuraria.*

2. An agent for increasing α-diversity of intestinal bacterial flora, comprising, as an active ingredient, a glycogen derived from *Galdieria sulphuraria.*

3. An agent for increasing a proportion of bifidobacteria in an intestinal bacterial flora, comprising, as an active ingredient, a glycogen derived from *Galdieria sulphuraria.*

4. An agent for increasing a proportion of butyric acid bacteria in an intestinal bacterial flora, comprising, as an active ingredient, a glycogen derived from *Galdieria sulphuraria.*

5. The agent according to claim 4, wherein the butyric acid bacteria are at least one selected from the group consisting of bacteria belonging to the genus *Coprococcus* and bacteria belonging to the genus *Blautia.*
